# EUROPEAN PATENT SPECIFICATION

(11) **EP 2 088 955 B1**
(45) Date of publication and mention of the grant of the patent: **16.02.2011**
(21) Application number: 07818662.4
(22) Date of filing: 03.10.2007
(51) Int. Cl.: A61B 19/00, F16C 11/10, F16M 11/14, A61B 17/00, A61B 17/02

(54) **LOCKABLE JOINT**
ARRETIERBARES GELENK
JOINT VERROUILLABLE

(30) Priority: 06.10.2006 US 850090 P; 29.05.2007 US 932127 P; 29.06.2007 US 963699 P
(43) Date of publication of application: 19.08.2009
(73) Proprietor: Fricke, Helmut, 38536 Meinersen (DE)
(72) Inventor: Fricke, Helmut, 38536 Meinersen (DE); Griffith, Lawrence, E., Lakeville, MN 55044 (US)
(74) Representative: Plöger, Jan Manfred
(86) International application number: PCT/EP2007/008582
(87) International publication number: WO 2008/040537

(56) References cited:
- EP-A- 1 520 548
- US-A- 5 201 325
- US-A1- 2006 069 395
- US-B1- 6 632 170

## Description

### Field of the Invention

The invention relates generally to the field of surgical instrumentation. More particularly, the invention relates to a lockable joint, especially a lockable ball-and-socket joint, comprising a first arm, the first arm having a swivel head, a socket, the swivel head being pivotably mounted to the socket, a locking device arranged for looking the swivel head with respect to the socket, the locking device having a piston, a pressure gas source, and an actuating device arranged for reversibly disconnecting the piston from the pressure gas source. The invention also relates to the field of related structures which are secured to a surgical table or other fixed structure.

Such lockable joints are known from US 5,201,325, US 6,632,170, US 5,201,325, and EP 1 520 548 A2. The lockable joints in these documents can be actuated by pressured air that is delivered via a gas hose. As the gas pressure of pressurized gas in gas hoses usually is not sufficient to clamp the lockable joint safely, a pressure enhancing system is provided. Another lockable joint is known from US 5,271,384. This lockable joint suffers from a clamping force that is not sufficient for many applications.

It is the problem of the present invention to enhance the reliability and the clamping force of the joint.

The invention solves the problem with a lockable joint, especially a lockable ball-and-socket joint, comprising a first arm, the first arm having a swivel head, a socket, the swivel head being pivotably mounted to the socket, a locking device arranged for locking the swivel head with respect to the socket, the locking device having a piston, a pressure gas source, and an actuating device arranged for reversibly disconnecting the piston from the pressure gas source wherein the pressure gas source is a gas cartridge.

According to another aspect, the invention solves the problem with a lockable joint, especially a lockable ball-and-socket joint, comprising (a) a first arm, the first arm having a swivel head, (b) a socket, the swivel head being pivotably mounted to the socket, and (c) a locking device arranged for locking the swivel head with respect to the socket, the locking device having (i) a piston, (ii) a gas cartridge retainer arranged for removably receiving a gas cartridge, and (iii) an actuating device arranged for reversibly disconnecting the piston from the gas cartridge or gas cartridge retainer.

According to a still further aspect, the invention solves the problem with a method for fixing a lockable joint according to claim 27.

### Background of the Invention

Usually, surgical retractors were hand-held instruments with multiple curved fingers used to hold open incisions during surgical procedures. The surgeon or an assistant would hook the fingers of the surgical retractor over the edge of an incision and apply tension to hold the incision open to provide access for the surgeon to internal bodily structures.

In approximately the last two decades, surgical retractors have been developed that are secured to a surgical table or other structure to allow retraction to be accomplished without the necessity of the surgeon or an assistant constantly holding the retractor.

For surgical retractors system according to the invention, a table rail post may be the foundation of a surgical retractor system. It provides an anchor for a frameset and other hardware onto which retractor instruments and other surgical instruments may be attached. A variety of retractor instruments with variably shaped retractor fingers are used in surgery to assist the surgeon in holding a surgical incision open or to move anatomical structures out of the way. The surgical retractor systems may use cam mechanisms or occasionally screw clamps to lock various members of the retractor system in position.

The surgical retractor system according to the invention may be a round stock retractor system and/or flat stock retractor system. Flat stock retractor systems suffer the limitation that because of the rectilinear nature of the various components, the components must be joined at substantially right angles in order to interconnect. Thus, the number of orientations in which flat stock retractor systems can be assembled is limited.

Round stock retractor systems generally are preferred because they allow for the interconnection of the various retractor system components at a variety of different angles because of the ability of the round stock parts of the system to rotate relative to one another and to clamp components.

Round stock retractor systems include various rod shaped parts that, initially, are connected together by screw-threaded type clamps. When screw-threaded type clamps are used, there might be a tendency for the screw clamps to deform the cylindrical members of the retractor system. Further, setting up, positioning and interconnecting the parts of the retractor system can require both hands, or possibly both hands of one individual, plus an assistant to assemble the system. Thus, cam lock or over center lock connection systems are preferred for mounting the surgical retractor to the first arm and/or for fixing the anchor element to the third arm.

The cam lock system may include two interconnected clamps that are configured to grip the rod shape retractor system members and that can be adjusted in rotation relative to each other. One rod shaped component is gripped in each clamp. The two interconnected clamps are activated by some sort of actuator such as a lever which then locks the two clamps to two rod-shaped members and also simultaneously locks the two camps relative to each other in rotation. One disadvantage of this arrangement is that when the clamps are released, they are released completely from both rod-shaped members as well as in relative rotation, requiring that the retractor system be completely repositioned and realigned before re-clamping.

Surgical retractor systems are used to manipulate living tissue. The application of pressure to living tissue can damage cell structure or reduce blood flow to the tissue. Living tissue can be damaged by the application of pressure for too long a time. Therefore, it is recommended that during surgical procedures where mechanical retractors are used, periodically the retractors should be loosened or tension should be lessened on the retractors to allow increased blood flow to the tissue being retracted to prevent tissue hypoxia and possible necrosis. This requirement, along with the limitation of current retractor systems, creates a dilemma for the surgical team. The surgical teams can disconnect the surgical retraction system periodically but then be required to make complete adjustments of each surgical retractor to reconnect it. Alternately, the surgical team can leave the living tissue retracted under tension for long periods of time and risk tissue damage or necrosis to the tissues being retracted. Surgical team members tend to be reluctant to disconnect and then readjust the retractor system if the readjustment is time consuming or unwieldy or if readjustment will alter the carefully positioned relationship of anatomical structures.

Another issue that arises with current round stock surgical retractor systems arises from the fact that surgeons generally prefer to locate retractors so that they are providing retractive tension at an angle. Surgeons prefer this approach in order to move the retractor to one side of the field in which they must work so that the retractor does not interfere with their movements. When the surgical retractors are offset, quite often it is impossible to position the retractors so that the retractor is pulling completely linearly with relation to the long axis of the rod-shaped members. This imparts a torsional or rotational force to the clamps that are secured to cylindrical or rod shaped members of the retraction system. This force tends to cause the clamps of the retraction system to slip about the rods in a rotational fashion. A common response to this problem is to increasingly tighten the clamp that is applied to the rod-shaped member. Unfortunately, when tightened beyond a certain point, the clamp will tend to create deformation or galling of the rod shaped member to which it is clamped making it more difficult to adjust the system for future usage.

A problem that arises with currently available retraction systems is that when a retractor is fixed to the system by a current clamp the multi-axis joint created between components is completely locked so that the components joined are immovably fixed in all axes. Commonly, it is necessary for the surgeon or an assistant to increase or readjust retractions for certain activities. Adjusting retractions means that the surgeon or an assistant must loosen the clamp holding the retractor, reposition the retractor, and then reapply the clamp. Since prior art clamp releases completely from two rods and in rotation simultaneously, at least two hands are required to realign and retighten the system. This can be quite awkward as there is a period of time where tension on the retractor is reduced and tissues may move in an undesirable fashion when the tension is reduced.

Retractor frames generally include a first frame arm, a second frame arm and a locking device that may also be called clamping member and that secures the left frame arm and the right frame arm in a fixed position, so that a surgical retractor may secured to the left and right frame arms. In addition, retractor frames generally include a third support arm which can be secured to a surgical table rail post. Existing retractor frames suffer a number of limitations. For example, the clamping member that secures the right and left frame arms generally locks the right and left frame arms in position simultaneously. While convenient locking, the frame arms simultaneously can make it difficult to adjust the right and left frame arms independently of one another. In addition, in many prior art retractor frames the clamping member also secures the pivotable connection between the support arm and the claming member at the same time that the right and left frame arms are secured, sometimes making it difficult to adjust the retractor frame as desired.

### Summary of the Invention

For the purpose of the following description a locking device may be any device that is arranged and adapted for immobilizing the swivel head relative to the socket. It is possible to provide two or even more locking devices. The locking device may provide a frictional locking with the swivel head. Alternatively, the locking device may be adapted for a positive locking or for a combination of both positive and frictional locking.

The actuating device may be any device that is adapted and arranged for allowing and interrupting a gas communication between the gas cartridge and the piston. For example, the actuating device may be a valve.

A gas cartridge may be a cartridge that contains a chemical composition or mixture of chemical compositions that is/are gaseous at ambient temperature of 20°C and ambient air pressure of 1013 hPa. Alternatively, the gas cartridge contains a chemical composition that may be brought into contact with another chemical composition to react chemically so that such a gas is produced.

It is an advantage of the present invention that it is not necessary to provide an external source of pressurized air. Such an external source of pressurized air may not be available. Especially in hospitals, pressurized air systems are often contaminated with bacteria, so that this air must not be used for operation devices. As a further advantage, air hoses are no longer needed. Air hoses bear the risk of bursting and have to be checked regularly. They are also inconvenient to use and bear the risk of stumbling for personnel. It is another advantage that the gas cartridge may be a disposable gas cartridge. Disposable gas cartridges are easy to handle and to store, so that the lockable joint has a high reliability and availability. It is another advantage that the lockable joint can be actuated easily. A lockable joint is therefore advantageous for high precession applications.

In a preferred embodiment, that gas cartridge has an internal pressure of more than 4 MPa or 40 bar. In particular, the internal pressure is more than 5 MPa or 50 bar. This high pressure makes it possible to actuate the locking device directly by the piston. Standard air pressure systems used e.g. in hospitals usually have a pressure of below 0,7 MPa, so that the force of the piston has to be increased by a suitable device. These devices are error-prone and expensive. The use of a gas cartridge having an internal pressure of more than 4 PMa thus leads a lockable joint that is easy to manufacture, cheap and robust.

In a preferred embodiment, the gas cartridge contains less than 1000 g of gas. In particular, the gas cartridge contains less than 100 g of gas, e.g. 12 g to 50 g of gas. These kinds of gas cartridges are small and easy to handle, but contain a sufficient amount of gas for most applications of the lockable joint.

It is preferred that the gas cartridge contains carbon dioxide, pressurized air, pressurized nitrogen, pressurized nitrous oxide, pressurized noble gas, pressurized oxygen, or a mixture of two, three, four or five of the aforementioned substances. In particular, it is preferred that the gas cartridge contains carbon dioxide and may therefore be called a carbon dioxide cartridge. Carbon dioxide is non-toxic, easy to manufacture and harmless to handle. It is an advantage, that carbon dioxide is a liquid at room temperature of 20°C, if the pressure is above 5,8 MPa. As a liquid, carbon dioxide has a high density so that even small carbon dioxide cartridge can store a significant amount of carbon dioxide.

It is preferred that the carbon dioxide cartridge contains sterile carbon dioxide. Used carbon dioxide from a sterile carbon dioxide cartridge may be released into the ambient air even in an operating room.

It is preferred that the lockable joint has a main body, the main body comprising the socket in the locking device, the locking device having a fixing element that is received in the main body, and the piston being arranged for pressing the fixing element against the swivel head. It is particularly advantageous that the piston and the fixing element are connected, such that moving the piston in a piston working direction leads to movement of the fixing element in a fixing working direction, whereby the piston working direction is parallel to the fixing element working direction. This yields a particularly advantageous flux of force.

It is a particular advantage if the piston and the fixing element are directly coupled, such that moving the piston by pre-determined pistons leads to a movement of the fixing element by the same pre-determined distance. That is, no force increasing mechanism is required. That leads to an easy to manufacture lockable joint. In particular, the piston is arranged for pressing against the swivel head. The contacting part may be called fixing section.

In an preferred embodiment, the main body comprises a cylinder, the cylinder having an inlet opening, the piston being moveably received in the cylinder and comprising a fixing section for pressing against the swivel head, the fixing section being located opposite the inlet opening. In this embodiment, gas streaming thru the inlet opening into the cylinder pushes the piston towards the swivel head thus pressing its fixing section against it. The socket is arranged such that the swivel head presses against the socket when the fixing section presses against the swivel head. Thus, the swivel head is locked between the socket and the fixing section. As the piston is snugly received in the cylinder and as the socket is rigidly mounted to the main body, the swivel head is locked, i.e. tightly fixed and clamped, to the main body. If a high gas pressure is provided, e.g. 6MPa, a clamping force of more than 4000 N is easily achievable.

In a preferred embodiment, the main body comprises a gas cartridge retainer for changeably receiving the gas cartridge. It is then possible to use disposable or one-way gas cartridges that are easily available at low prices. Further, it is possible to use the lockable joint in places, where a pressurized air system is not available. Examples are developing countries or remote places.

It is preferred that the fixing element, in particular the fixing section of a piston, is arranged for contacting the swivel head in a fixing element contact area that is a ring-shaped and has a ring width of less than 1 mm. This yields a particularly strong clamping force that the locking device exerts on the swivel head, as a small ring width leads to a high pressure that in turn causes an elastic deformation of the swivel head.

In a preferred embodiment, the fixing element is arranged for frictional locking between the fixing element and the swivel head. This leads to an easy to manufacture and robust lockable joint.

The contact section area may be segmented. To achieve this, the fixing element, e.g. the fixing section of the piston, may be provided with clearances or cuts. These clearances may also be arranged to enable cleaning or the lockable joint, in particular the swivel head, after use or to disinfect the lockable joint.

It is preferred that the swivel head has swivel head outer diameter and contacts the fixing element, e.g. the fixing section of the piston, in a ring-shaped swivel head contact area that is larger than 0,94 times the swivel head outer diameter.

It is preferred that the first arm has a arm longitudinal axis and the contact area outer diameter and the arm longitudinal axis form a substantially constant effective angle or flash than 20°. In particular, it is preferred that the effective angle is larger than 1°.

To allow for as many lock-and-release cycles, it is preferred that the gas cartridge has a gas cartridge outlet opening, the cylinder and a gas cartridge being in gas communication via a gas conduct between the gas cartridge outlet opening and the cylinder inlet opening, wherein the gas conduct has an volume of less than 1000 mm³, in particular less than 500 mm³. To maximize the number of lock-and-release cycles, it is also preferred that the piston has a stroke of less than 2 mm, in particular less than 1 mm.

To provide for a strong clamping force, the piston preferably has a piston diameter of more than 20 mm.

According to another aspect, the invention relates to a lockable joint assembly that comprises (a) a lockable joint according to the first aspect of the invention, (b) a second arm having a second swivel head, (c) a second socket head being pivotably mounted to the second socket, (d) a second locking device arranged for locking the second swivel head with respect to the second socket, the second locking device having (i) a second piston disconnectably connected to the gas cartridge and (ii) a second actuating device arranged for reversibly disconnecting the second piston from the gas cartridge. For the sake of easy handling, the second arm is preferably arranged opposite the first arm.

To fix the lockable joint to an anchor element, the lockable joint preferably comprises (a) a support arm having a support swivel head, (b) a support arm socket, pivotably mounted to the support arm socket, and (c) a support arm locking device arranged for locking the support arm swivel head relative to the support arm socket, the support arm locking device having (i) a support arm piston interruptible connected to the gas cartridge and (ii) a support arm actuating device arranged for reversibly disconnecting the support arm piston from the gas cartridge.

According to still a further aspect, the invention relates to a surgical retractor system, comprising (a) a lockable joint assembly according to the invention, (b) an anchor element adapted for mounting to an operating table, the anchor element being releasable mounted to the support arm, the first arm being adapted to act as a frame element for receiving at least one surgical retractor.

The surgical retractor frame of the present invention is adapted to be anchored to a surgical post secured to a surgical table rail, or to another fixed structure, to allow the application of surgical retractors that are used for the retraction of anatomical structures.

The surgical retractor frame of the present invention may include a main body in form of a control hand piece, a support arm and two frame arms, i.e. the first arm and the second arm.

The support arm and two frame arms (first arm and second arm) can be articulated with the control hand piece via ball joints. Each ball joint supports one of the support arms and the two frame arms. Each ball joint is independent lockable and releasable at any location within its articular range. That is, each ball joint can be independently released, adjusted and secured independent of the other two ball joints.

The independent gas pressure driven locking mechanism of the present invention is operated by a pressurized gas source in form of gas cartridge, e.g. a small pressurized gas cylinder containing pressurized gas such as carbon dioxide, nitrogen, or compressed air.

In one exemplary embodiment, the pressurized gas supply is provided in a small carbon dioxide cartridge or cylinder. The carbon dioxide cartridge is contained within the control hand piece of the present invention. The carbon dioxide cylinder is intended to be replaced with each use of the surgical retractor frame. For example, the control hand piece may have a generally cylindrical chamber into which the compressed gas cylinder may be placed. Once the gas cylinder is placed in the chamber, a screw for carding the gas cylinder may be inserted behind the gas cylinder and tightened until the gas cylinder is pierced by a trocar or hollow needle. The gas cylinder is simultaneously sealed to the control hand piece.

The main body or control hand piece may further include an independent push button or other valve actuator to operate each individual spherical ball joint. The support arm locking device operates the ball joint for the support arm.

In one aspect of the invention, the operation of the support arm locking device is such that the support arm is locked by pressurized gas pressing the support arm piston against the swivel head, i.e. a spherical member, of the ball joint except when the actuating device, e.g. a push button, is depressed. The support arm locking device is structured such that when its push button is depressed, pressurized gas acting against the support arm piston is released while pressurized gas is sealed off within the gas cartridge so that gas is not vented constantly. The actuating device for the first arm operates the first arm, i.e. the right frame arm, and the second locking device operates the second arm, i.e. the left frame arm. The ball joints are arranged so that some tension is kept on the spherical member by the piston, even when the gas pressure is released. This provides tension so that the frame arm may be adjusted into a desired position without "flopping" in response to gravity.

In one aspect of the invention, the pressurized gas cylinder has a fail-safe pressing release so that if a hospital staffer mistakenly places the surgical retractor frame in an autoclave for sterilization with the gas cylinder still installed, the gas cylinder will vent safely, thus preventing the risk of an explosion in the autoclave.

In one aspect of the invention, the pistons which bear with their fixing sections against the ball joint spheres have a piston seal including a stainless steel spring within the piston seal. O-rings or pistons without seals may also be utilized.

The compressed gas that is used to operate the pistons in the surgical refractor frame may be supplied at a pressure between about 200 and 350 pounds per square inch. In one aspect of the invention, the system operates at pressures of roughly 300 pounds per square inch.

It is notable that the piston travel in the surgical retractor frame may be very short; on the order of less than one millimeter. The fit of the piston within the cylinder is quite tight so that the piston maintains tension on the sphere of the ball joint even when the pressure on the piston is released. The piston and sphere of the ball joint may be made of a metallic material such as, for example, Nitronic 60, Galltough™, or V4A^{™} steel.

The locking devices may comprise dual function push button valves that both vent pressure from the respective piston and cylinder and seal the gas passages or ducts from the pressurized gas source simultaneously.

The control hand piece may include an ergonomic hand grip. The body of the control hand piece may be formed of, for example, stainless steel such as 400 series stainless steel or V4A steel.

In one embodiment of the invention, the first and second, e.g. the left and right, frame arms and the support arm may be tubular structures rather than the more conventional solid bars. This lightens the structure of the surgical retractor frame without significantly sacrificing strength. The spheres of the ball joints may be solid or hollow. All of the metallic parts of the present invention may also be formed of materials such as titanium or stainless steel.

The gas cartridge may be supplied in a sterile or a non-sterile state. When non-sterile cartridges are used, a sterile sleeve may be used to maintain a sterile field in the operating room to allow for cartridge exchange, if needed, during a surgical procedure.

Preferred embodiments are now described with respect to the drawing, in which
- Figure 1: is a perspective view of an exemplary embodiment of a surgical retractor system according to the present invention;
- Figure 2: is a perspective view of an exemplary embodiment of a lockable joint assembly according to the present invention;
- Figure 3a: is a side cross sectional view according to plane A of the embodiment depicted in Figure 2 in a locking position;
- Figure 3b: is a schematic cross sectional view of a detail of figure 3a;
- Figure 3c: is a view according to figure 3a depicting a release position;
- Figure 4a: is a first front cross sectional view of an alternative embodiment of a lockable joint assembly according to the present invention;
- Figure 4b: is a second front cross sectional view of the embodiment of figure 4a;
- Figure 5a: is a perspective view of the piston comprising the fixing element;
- Figure 5b: is a side cross sectional view of the piston of figure 5a;
- Figure 5c: is a front view of the piston depicted in figures 5a and 5b;
- Figure 6a: a socket for a lockable joint according to the invention in a perspective view;
- Figure 6b: a cross sectional view of the socket of figure 6a;
- Figure 6c: is a side view of the socket of figures 6a and 6b;
- Figure 6d: a cut along C-C of Figure 6c;
- Figure 7: is a perspective view of a support arm and two frame arms of a surgical retractor frame in accordance with the invention;
- Figure 8: is a cross sectional view of another embodiment of a lockable joint in accordance with the invention; and
- Figure 9: is a cross sectional view of still another embodiment of a lockable joint in accordance with the invention.

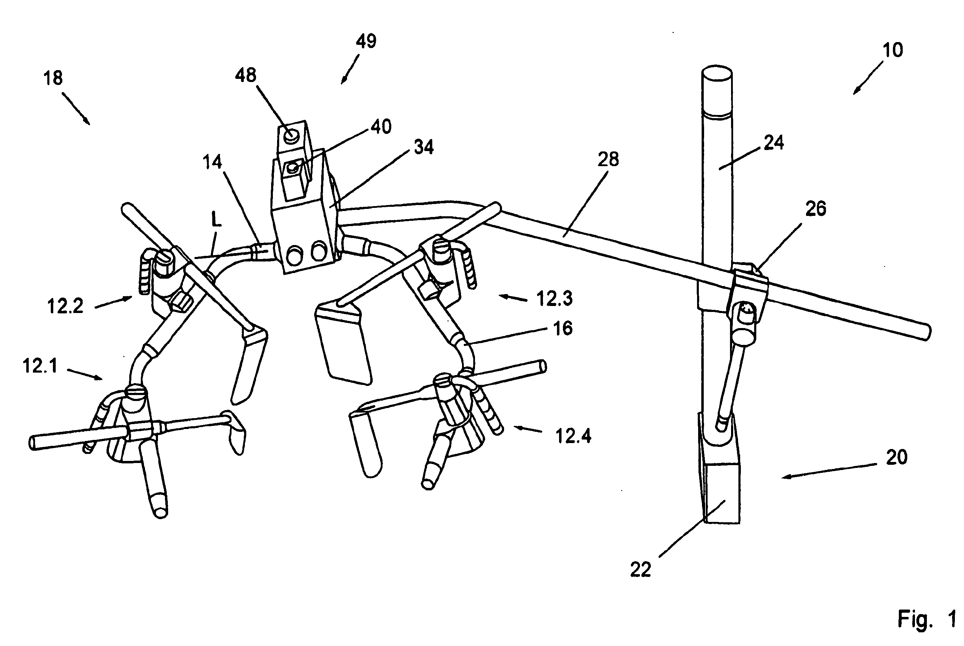

Figure 1 shows a surgical retractor system 10 comprising four surgical retractors 12.1, 12.2, 12.3 and 12.4. In the following, reference numerals without counting suffix refer to the object as such. Surgical retractors 12 are mechanically clamped either to a first arm 14 or a second arm 16 of a surgical retractor frame 18.

The surgical retractor frame 18 also comprises an anchor element 20 that is adapted for mounting to an operating table (not shown). Anchor element 20 has a coupling anchor 22 that is connected to a clamping rod 24. An anchor element joint 26 is provided to releasably and pivotably mount a support arm 28 of the surgical retractor frame 18. First arm 14 and second arm 16 have the same shape and are sectionwise cylindrical and form a bow. Support arm 28, first arm 14, second arm 16 and a schematically shown main body 34 (described below in greater detail) linking them to each other are parts of a lockable joint assembly 49 (also described below in greater detail).

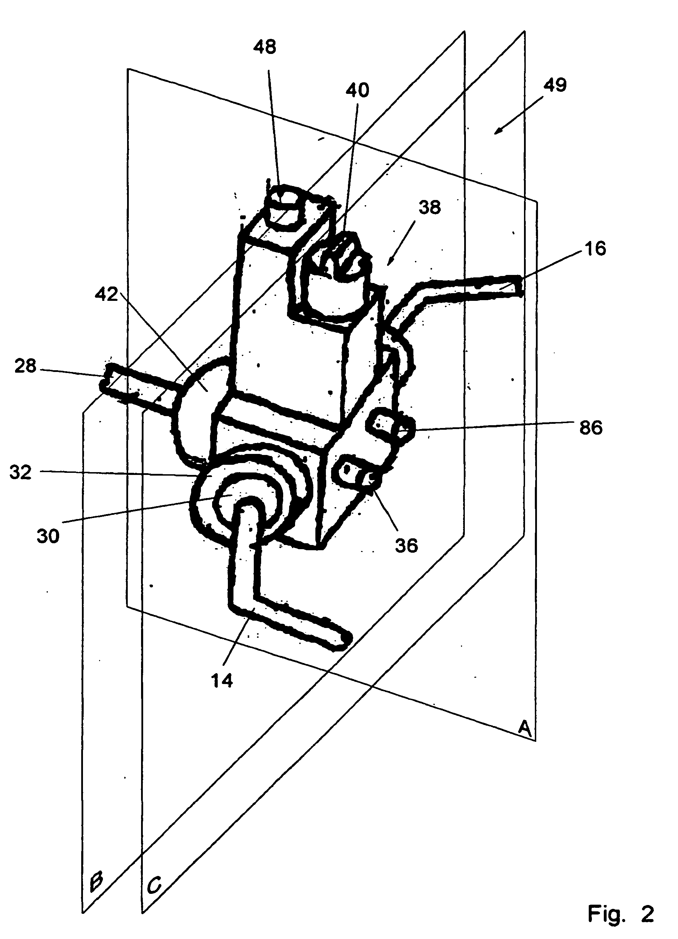

Figure 2 depicts lockable joint assembly 49. First arm 14 has a swivel head 30 that is received in a socket 32. Socket 32 is part of main body 34 that may also be called a control hand piece. Main body 34 comprises a push button 36 of a locking device that comprises components that are used to lock or release first arm 14 to main body 34 and will be described in greater detail below. Main body or control hand piece 34 also comprises a gas cartridge retainer 38 that has a gas cartridge chamber screw 40, which is arranged for releasably . Figure 2 also depicts a support arm socket 42 that is also part of main body 34.

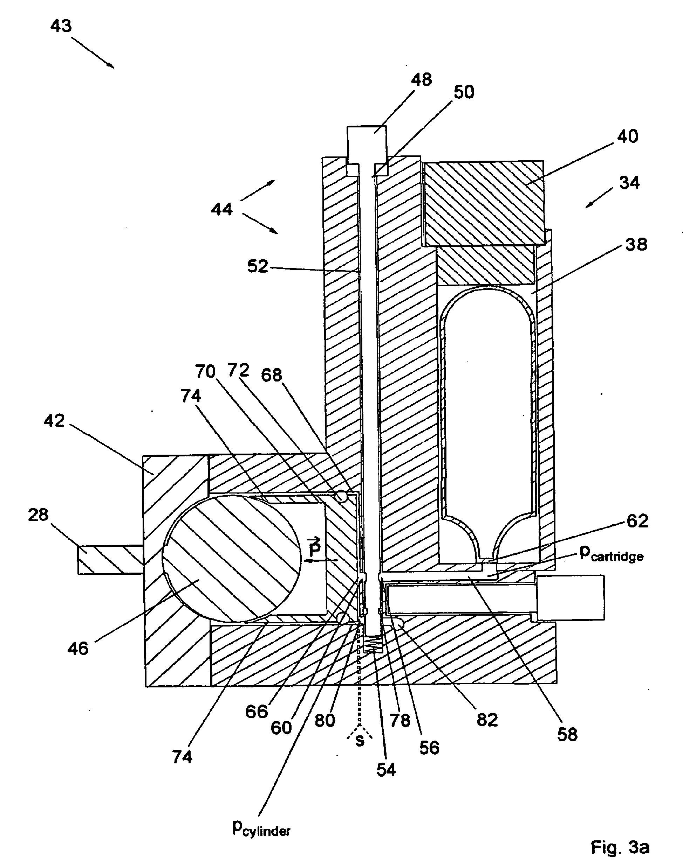

Figure 3a shows a sectional view of a lockable joint 43 according to the invention. At the same time, figure 3a depicts a cross section of lockable joint assembly 49 with respect to plane A of figure 2. It is to be noted that a lockable joint 43 by definition has only one arm and one joint, especially one ball joint. Devices with more than one arm are referred to as lockable joint assemblies. Preferably, all arms of the lockable joint assembly are mounted to one single rigid main body. Thus, a lockable joint 43 is part of lockable joint assembly 49.

In figure 3a a support arm locking device 44 is depicted that is adapted for releasably locking a support arm swivel head 46 of support arm 28 to main body or control hand piece 34. Support arm locking device 44 comprises a support arm push bottom 48 that protrudes over main body 34, such that at it can be easily actuated by finger tip. Alternatively, support arm push button 48 may be completely located in a recess, so that it is protected against unintended actuation.

Support arm locking device 44 also comprises a support arm valve member 50 that has a substantially cylindrical shape and is snugly received in bore 52 and sealed in a gas-tight against it. Support arm valve member 50 is mounted to support arm push button 48 and is pre-tensioned in a locking position (shown in Figure 3a) by a spring 54 located opposite the support arm push button 48.

In the locking position, an annular recess 56 in support arm valve member 50 provides gas communication of a first gas channel 58 to a second gas channel 60 that both have a diameter of 3 mm. Annular recess 56, first gas channel 58, and second gas channel 60 form a gas duct 61 having a volume V. This volume V should be reasonably small, e.g. 250 mm³ or less. However, a larger volume V is possible as well. First gas channel 58 may and extends from bore 52 to gas cartridge retainer 38 and in particular to a gas cartridge outlet opening 62 of a carbon dioxide cartridge 64. Carbon dioxide cartridge 64 may be disinfected e.g. by x-ray or γ-ray exposure.

Carbon dioxide cartridge 64 contains about 12 grams of carbon dioxide at a pressure P_{cartridge} of about 6 MPa. At room temperature, most of the carbon dioxide is liquid due to the high pressure. Carbon dioxide escapes from carbon dioxide cartridge 64 through first gas channel 58, annular recess 56, and second gas channel 60 and streams through a cylinder inlet opening 66 into a cylinder 68. In cylinder 68, a support arm piston 70 is received and sealed in gas-tight manner via an o-ring 72. Alternatively, a gasket or washer may be used.

In the locking position shown in Figure 3a, a gas pressure P_{cylinder} in cylinder 68 equals to the gas pressure p_{cartridge} in carbon dioxide cartridge 64. Thus, piston 70 is pressed in a piston working direction P̅ so that a fixing section 74 of cylinder 68 that acts as a fixing element is pressed against support arm swivel head 46. In other words, piston 70 presses directly against the swivel head. In figure 3a, piston working direction P̅ equals to a fixing element working direction. The distance between a locking position of support arm piston 70 and a position in which it is completely retracted, i.e. in which it abuts the wall with cylinder inlet opening 66, is called stroke s. In other words, stroke s is the maximum piston travel. It equals 0,5 mm.

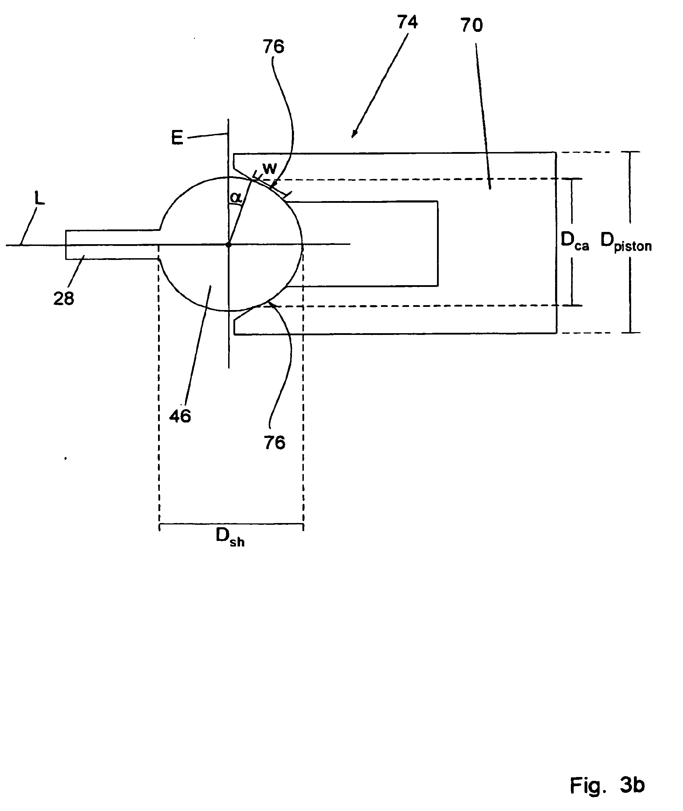

Figure 3b shows a schematic cross section of support arm piston 70 and support arm swivel head 46. The fixing element in form of fixing section 74 contacts the support arm swivel head 46 in a fixing element contact area 76. The fixing element contact area 76 is ring-shaped and has a ring width w of about 0,5 mm. The surfaces of support arm swivel head 46 and fixing section 74 are polished or grinded so that a frictional locking is provided. Support arm swivel head 46 is a ball or a sphere and has a swivel head outer diameter Dₛₕ. Support arm piston 70 has a diameter Dₚᵢₛₜₒₙ, that is 0,94 times the swivel head of the diameter Dₛₕ or larger. Contact area 76 has a contact area outer diameter D_{ca} that 0,94 times the swivel head of the diameter Dₛₕ or larger.

Support arm 28 has a longitudinal axis L. If longitudinal axis L is aligned with the longitudinal axis of support arm piston 70, as shown in figure 3b, an effective angle α is formed between a plane E perpendicular to longitudinal axis L and contact area outer diameter D_{ca}.

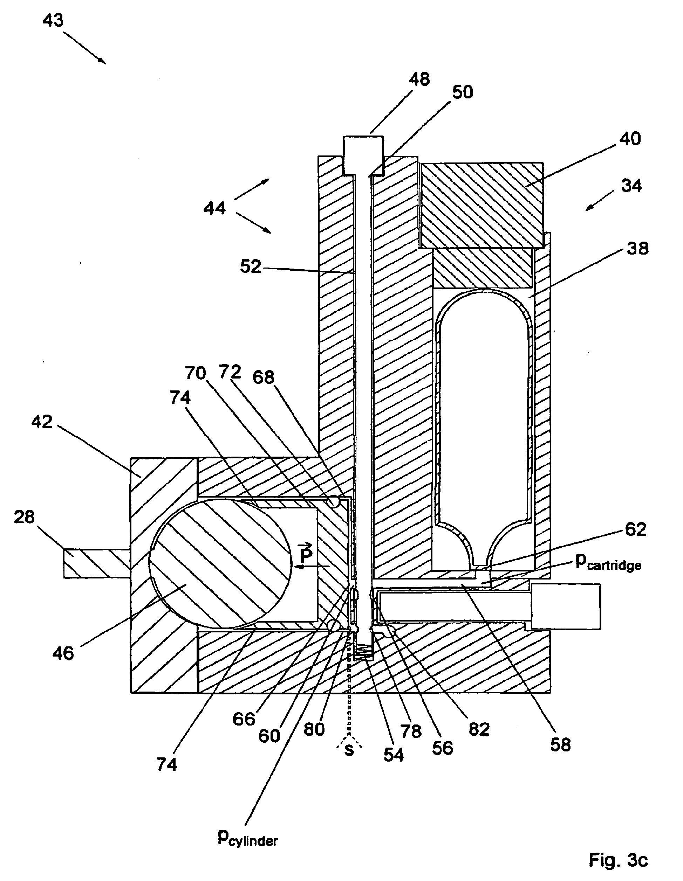

Figure 3c shows support arm locking device 44 in its release position with support arm push button 48 pushed down against the biasing force of spring 54. It can be seen that annular recess 56 no longer links first gas channel 58 to second gas channel 60 thus interrupting cylinder 68 from carbon dioxide cartridge 64. In the release position, a second annular recess 78 connects a first outlet channel 80 to a second outlet channel 82 that leads to an exhaust opening (not shown). Thus, in the release position, carbon dioxide escapes through first outlet channel 80, second annular recess 78, and second outlet channel 82 until the gas pressure p_{cylinder} in cylinder 68 equals the ambient air pressure p_{ambient}. Support arm swivel head 46 may now be pivoted freely with respect to main body 34.

First arm 14 and second arm 16 each have a respective swivel heads cooperating with a respective piston as described above for support arms swivel head 46 and support arm piston 70. Push button 36 (figure 2) is used to release first arm 16 and a second push button 86 is used two release second arm 16.

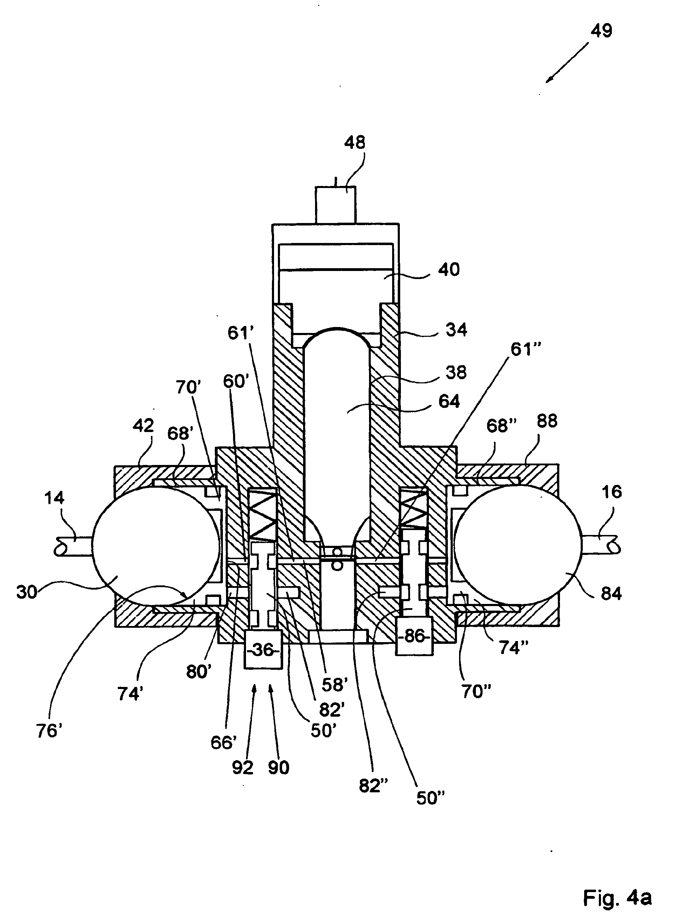

Figure 4a shows a cross section according to plane B of Figure 2 for a second embodiment of the lockable joint assembly 49. In Figure 4a push button 36 and second push button 86 are located at the downside of main body 34. This embodiment is chosen to ease the understanding of the mechanism. However, the embodiment shown in Figure 2 has a respective structure. As shown in Figure 4a, second arm 16 has a second swivel head 84 that is received in a second socket 88.

Figure 4a also shows a locking device 90 for first arm 14, having a piston 68', the gas cartridge 64, and an actuating device 92. The actuating device 92 comprises push button 36 and valve member 50'. To avoid repetitions, similar elements as described above are referenced with like reference numerals having a slash added. For example, cylinder 68' has the same features as cylinder 68. In figure 4a, push button 36 is in its locking position with cylinder 68' in gas communication only with gas cartridge 64 and push button 86 is in its release position with cylinder 68" in gas communication only with the ambient air.

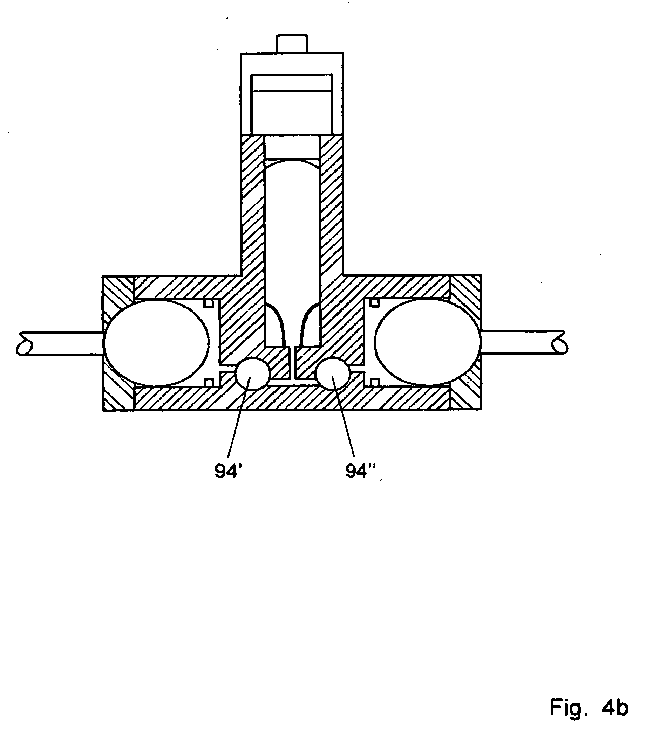

Figure 4b shows at a different level compared to figure 4a, i.e. with respect to a plane C that is parallel to plane B, but moved away from support arm 28 (see Figure 2). In figure 4b, two exhaust openings 94', 94" can be seen. Exhaust openings 94', 94" are in gas communication with second outlet channels 82', 82" (see figure 4a), respectively.

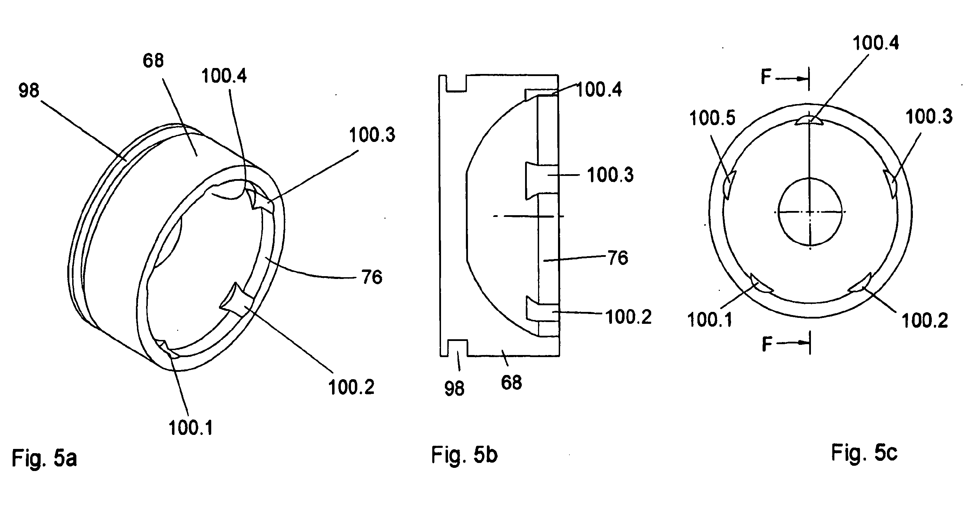

Figure 5a shows cylinder 68 having an annular recess 98 for o-ring 72 (not shown). Cylinder 68 has four clearances 100.1, 100.2, 100.3, 100.4. Due to these clearances, fixing element contact area 76 is segmented (see figure 3b).

Figure 5b shows a cross section of cylinder 68 and figure 5c shows a front view. Figure 5b is a cut along line F-F of figure 5c.

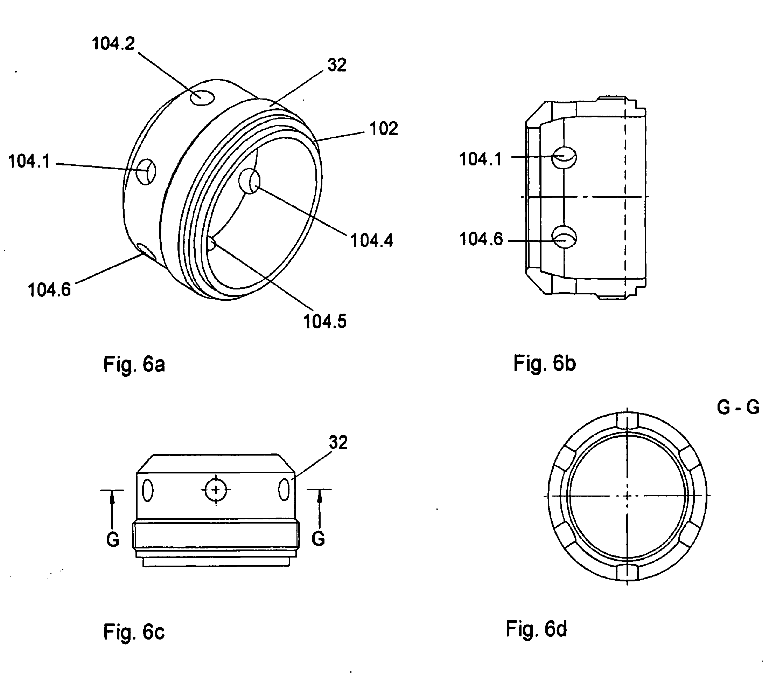

Figure 6a shows a perspective view of socket 32. Second socket 88 and support arm socket 42 have the identical shape. Socket 32 has a thread 102 for threaded engagement with a respective thread in main body 34 (see Figure 3a). Socket 32 has a plurality of bores 104.1, 104.2, ... that allow liquids or gases to reach those parts of swivel head 30, that are captured within socket 32. This is particularly advantageous for cleaning and disinfecting.

Figure 6b shows a cross section of socket 32, figure 6c is a side view, and figure 6d is a cut along line G-G of figure 6c.

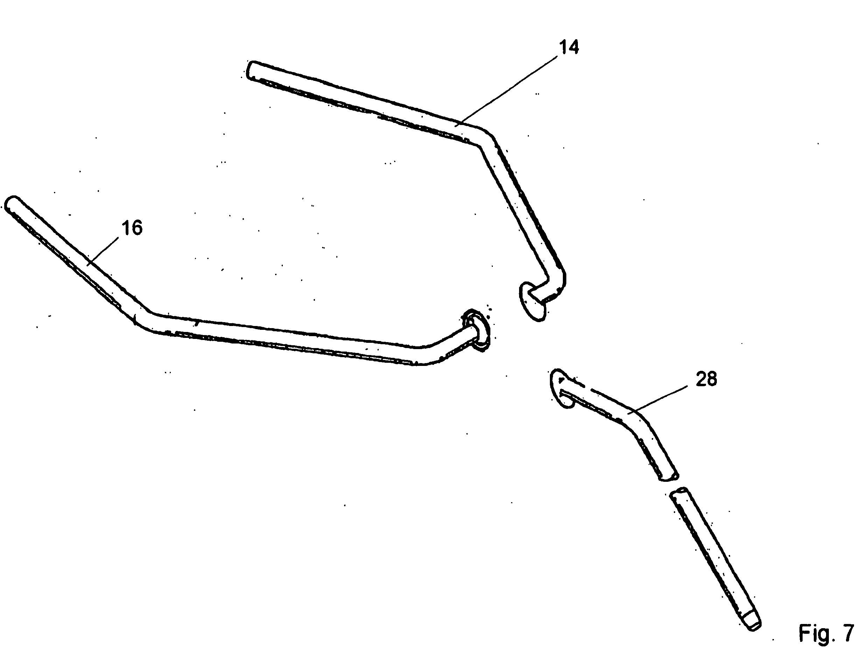

Figure 7 shows first arm 14, second arm 16, and support arm 28 in a prospective view.

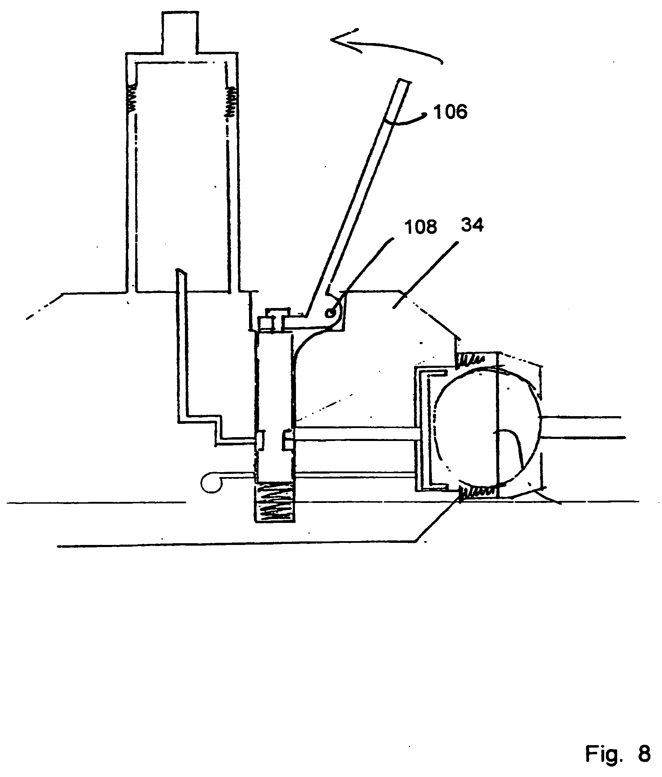

Figure 8 shows an alternative embodiment of an inventive lockable joint 43. For the sake of easy handling, a push button has been replaced by a lever 106 that is pivotably connected to main body 34 via a hinge 108.

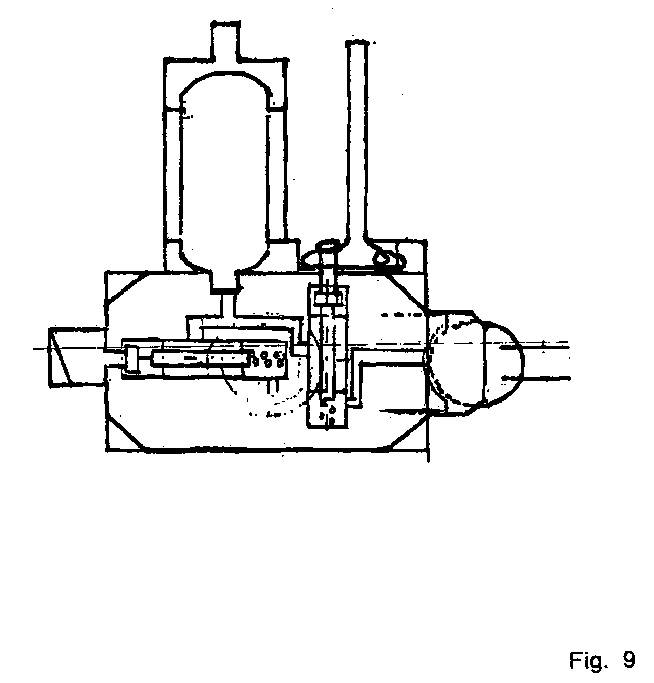

Figure 9 shows an embodiment of an inventive lockable joint assembly 49 in a cross sectional view.

Referring to Figure 3a and 4a, compressed gas cylinder 64 may be located within control hand piece 34. Screw for carding 40 forces compressed gas cylinder 64 against a hollow piercing member (not shown) that pierces a malleable seal of the gas cartridge 64 and connects the interior of compressed gas cylinder 64 with gas conduits within or coupled to control hand piece 34. Gas flow is then controlled by push buttons or other valve actuators to selectively apply or release pressure on pistons to lock or unlock the ball joints.

The present invention is described herein, using the example of a surgical retractor frame. It should be understood that there are many other applications of the present invention both in a surgical and a non-surgical setting, both within and outside of health care where it is desirable to fix or lock articulated joints such as but not limited to ball joints. It should be understood that the invention maybe applied to other devices such as: Articulating arm joint holding devices including but not limited to: endoscopic scopeholder; endoscopic retractor holder, fan and other; endoscopic instrument/device holder; surgical screen holder; leg stirrups; and vertical/table post clamp devices. Articulating clamps/joints used in patient positioning; Wilson frame for spinal applications; OR tables; adjustment and locking mechanisms; orthopedic traction devices; and bone cement applicator.

### Reference numerals

- 10: surgical retractor system
- 12: surgical retractor
- 14: first arm
- 16: second arm
- 18: surgical retractor frame

- 20: anchor element
- 22: coupling anchor
- 24: clamping rod
- 26: anchor element joint
- 28: support arm

- 30: swivel head
- 32: socket
- 34: main body
- 36: push button
- 38: gas cartridge retainer

- 40: gas cartridge chamber screw
- 42: support arm socket
- 43: lockable joint
- 44: support arm locking device
- 46: support arm swivel head
- 48: support arm push button
- 49: lockable joint assembly

- 50: support arm valve member
- 52: bore
- 54: spring
- 56: annular recess
- 58: first gas channel

- 60: second gas channel
- 61: gas duct
- 62: gas cartridge outlet opening
- 64: carbon dioxide cartridge
- 66: cylinder inlet opening
- 68: cylinder

- 70: support arm piston
- 72: O-ring
- 74: fixing section
- 76: fixing element contact area
- 78: second annular recess

- 80: first outlet channel
- 82: second outlet channel
- 84: second swivel head
- 86: second push button
- 88: second socket

- 90: locking device
- 92: actuating device
- 94,96: exhaust opening
- 98: annular recess

- 100: clearance
- 102: thread
- 104: bore
- 106: lever
- 108: hinge

- A, B, C, E: plane
- d: contact area outer diameter
- Dₚᵢₛₜₒₙ: piston diameter
- Dₛₕ: swivel head outer diameter
- D_{ca}: contact area outer diameter
- L: longitudinal axis
- p: gas pressure
- P̅: piston working direction
- s: stroke
- V: Volume
- w: ring width

## Claims

1. A lockable joint, especially a lockable ball-and-socket joint, comprising
(a) a first arm (14) having a swivel head (30),
(b) a socket (32), the swivel head (30) being pivotably mounted to the socket (32), and
(c) a locking device (90) arranged for locking the swivel head (30) with respect to the socket (32), the locking device (90) having
(i) a piston (70'),
(ii) a pressure gas source (64), and
(iii) an actuating device (92) arranged for reversibly disconnecting the piston (70') from the pressure gas source (64),
**characterized in that**
(d) the pressure gas source is a gas cartridge (64).

2. The lockable joint according to claim 1, the gas cartridge (64) having an internal pressure (p_{cartridge}) of more than 4 MPa, in particular more than 6 MPa.

3. The lockable joint according to claim 1 or 2, the gas cartridge (64) containing less than 1000g of gas.

4. The lockable joint according to any of claims 1 to 3, wherein the gas cartridge (64) contains liquid carbon dioxide, pressurized air, pressurized nitrogen, pressurized nitrous oxide, or a mixture of two or three of them.

5. The lockable joint according to any preceding claim,
- the lockable joint (43) having a main body (34), the main body (34) comprising the socket (32) and the locking device (90),
- the locking device (90) having a fixing element (74) that is received in the main body (34), and
- the piston (70') being arranged for pressing the fixing element (74) against the swivel head (30).

6. The lockable joint according to claim 5, the piston and the fixing element (74) being connected, such that
- moving the piston (70') in a piston working direction (P̅) leads to a movement of the fixing element in a fixing element working direction (P̅).
- the piston working direction (P̅) being parallel to the fixing element working direction (P̅).

7. The lockable joint according to claim 5 to 6, the piston (70') and the fixing element (74) being directly coupled, such that moving the piston (70') by a predetermined distance leads to a movement of the fixing element by the same predetermined distance.

8. The lockable joint according to claims 5-7,
- the main body (34) comprising a cylinder (68'), the cylinder (68') having a cylinder inlet opening (66'),
- the piston (70') being movably received in the cylinder (68') and comprising the fixing element in form of a fixing section (74') for pressing against the swivel head (30), and
- the fixing section (74') being located opposite the cylinder inlet opening (66').

9. The lockable joint according to claims 5-7, the main body (34) comprising a gas cartridge retainer (38) for exchangeably receiving the gas cartridge (64).

10. The lockable joint according to any preceding claim,
- the fixing element (74') being arranged for pressing against the swivel head (30) a fixing element contact area (76'), and
- the fixing element contact area (76') being ring-shaped having a ring width of less than 1 mm.

11. The lockable joint according to any of claims 5 to 10, the fixing element (74') being arranged for frictional locking between the fixing element (74') and the swivel head (30).

12. The lockable joint according to any of claims 10 or 11, the fixing element contact area (76') being segmented.

13. The lockable joint according to claims 11 or 12,
- the swivel head having a swivel head outer diameter (Dₛₕ) and contacting the fixing element in a ring-shaped swivel head contact area, and
- the contact area having a contact area outer diameter (D_{ca}) that is larger than 0,94 times the swivel head outer diameter (Dₛₕ).

14. The lockable joint according to any preceding claim,
- the first arm having a arm longitudinal axis (L) and
- the contact area outer diameter (D_{ca}) and the arm longitudinal axis (L) forming a substantially constant effective angle (α) of less than 20°.

15. The lockable joint according to claim 8,
- the gas cartridge (64) having a gas cartridge outlet opening (62),
- the cylinder (68') and the gas cartridge being in gas communication via a gas duct (61') between the gas cartridge outlet opening (62) and the cylinder inlet opening (66'), and
- the gas duct (61') having a volume (V) of less than 1000 mm³, in particular less than 500 mm³.

16. The lockable joint according to any preceding claim, the piston (70') having a stroke (s) of less than 2 mm, in particular of less than 1 mm.

17. The lockable joint according to any preceding claim, the piston having a piston diameter (Dₚᵢₛₜₒₙ) of more than 20 mm.

18. A lockable joint assembly, comprising
(a) a lockable joint (43) according to any preceding claim,
(b) a second arm (16) having a second swivel head (84),
(c) a second socket (88), the second swivel head (84) being pivotably mounted to the second socket (88), and
(d) a second locking device arranged for locking the second swivel head (84) with respect to the second socket (88), the second locking device having
(i) a second piston (70") in disconnectable gas communication with the gas cartridge (64), and
(ii) a second actuating device (86, 50") arranged for reversibly disconnecting the second piston (70") from the pressure gas cartridge (64).

19. The lockable joint assembly according to claim 18, the second arm (16) being arranged opposite the first arm (14).

20. The lockable joint assembly according to any of claims 18 or 19, comprising
(a) a support arm (28) having a support arm swivel head (46),
(b) a support arm socket (42), the support arm swivel head (46) being pivotably mounted to the support arm socket (42),
(c) a support arm locking device (44) arrange for locking the support arm swivel head (46) with respect to the support arm socket (42), the support arm locking device (44) having
(i) a support arm piston (70) in disconnectable gas communication with the gas cartridge (64), and
(ii) a support arm actuating device (48, 50) arranged for reversibly disconnecting the support arm piston (70) from the gas cartridge (64).

21. Surgical retractor frame, comprising:
(a) a lockable joint assembly (49) according to any of claims 18 to 20,
(b) the first arm (14) being adapted to receive a surgical retractor (12), and
(c) an anchor element (20) for mounting the lockable joint assembly (49) to an operating table,
(d) the anchor element (20) being releasably mounted to the support arm (28).

22. Surgical retractor system, comprising the surgical retractor frame (18) according to claim 21 and at least one surgical retractor (12) releasably mounted to the first arm (14).

23. Method for fixing a lockable joint (43), especially lockable ball-and-socket joint, having
- a first arm (14), the first arm having a swivel head (30),
- a socket (32), the swivel head (30) being pivotably mounted to the socket (32),
- a locking device arranged for locking the swivel head with respect to the socket, the locking device having
a piston,
a gas cartridge, in particular a carbon dioxide cartridge, and an actuating device arranged for reversibly disconnecting the piston from the gas cartridge,
comprising the steps:
- actuating the actuating device, thus de-pressurizing the piston such that the first arm may be pivoted relative to the socket and
- releasing the actuating device, thus pressurizing the piston by bringing it into gas communication with the gas cartridge such that the locking device locks the swivel head with respect to the socket.

## Patentansprüche

1. Arretierbares Gelenk, insbesondere ein arretierbares Kugelgelenk, das folgendes aufweist:
(a) einen ersten Arm (14) mit einem Gelenkkopf (30),
(b) eine Gelenkpfanne (32), wobei der Gelenkkopf (30) drehbar in der Gelenkpfanne (32) eingesetzt ist, und
(c) eine Sperrvorrichtung (90), die zum Sperren des Gelenkkopfes (30) gegenüber der Gelenkpfanne (32) eingerichtet ist, wobei die Sperrvorrichtung (90) folgendes aufweist:
(i) einen Kolben (70'),
(ii) eine Druckgasquelle (64), und
(iii) eine Betätigungseinrichtung (92), die für ein schaltbares Trennen des Kolbens (70') von der Druckgasquelle (64) eingerichtet ist,
**dadurch gekennzeichnet, dass**
(d) die Druckgasquelle eine Gaskartusche (64) ist.

2. Arretierbares Gelenk gemäß Anspruch 1, **dadurch gekennzeichnet, dass** die Gaskartusche (64) einen Innendruck (p_{kartusche}) von mehr als 4 MPa, insbesondere mehr als 6 MPa, aufweist.

3. Arretierbares Gelenk gemäß Anspruch 1 oder 2, **dadurch gekennzeichnet, dass** die Gaskartusche (64) weniger als 1000g des Gases beinhaltet.

4. Arretierbares Gelenk gemäß einem der Ansprüche 1 bis 3, **dadurch gekennzeichnet, dass** die Gaskartusche (64) flüssiges Kohlendioxid, Druckluft, unter Druck stehenden Stickstoff, unter Druck stehende Stickoxide oder eine Mischung aus zwei oder drei von diesen beinhaltet.

5. Arretiebares Gelenk gemäß einem voranstehenden Anspruch, **dadurch gekennzeichnet, dass**
- das arretierbare Gelenk (43) einen Grundkörper (34) aufweist und der Grundkörper (34) die Gelenkpfanne (32) und die Sperrvorrichtung (90) aufweist,
- die Sperrvorrichtung (90) eine Halteeinrichtung (74) aufweist, die in dem Grundkörper (34) gehalten ist, und
- der Kolben (70') für ein Anpressen der Halteeinrichtung (74) gegen den Gelenkkopf ausbildet ist.

6. Arretierbares Gelenk gemäß Anspruch 5, **dadurch gekennzeichnet, dass** der Kolben und die Halteeinrichtung (74) derart verbunden sind, dass
- das Bewegen des Kolbens (70') in einer Kolbenarbeitsrichtung (*̅P̅*̅) zu einer Bewegung der Halteeinrichtung in einer Halteeinrichtung-Arbeitsrichtung (*̅P̅*̅) führt,
- die Kolben-Arbeitsrichtung (*̅P̅*̅) parallel zu der Halteeinrichtung-Arbeitsrichtung (*̅P̅*̅) ist.

7. Arretierbares Gelenk gemäß Anspruch 5 oder 6, **dadurch gekennzeichnet, dass** der Kolben (70') und die Halteeinrichtung (74) direkt gekoppelt sind, so dass das Bewegen des Kolbens (70') über einen zuvor festgelegten Abstand zu einer Bewegung der Halteeinrichtung über den gleichen zuvor festgelegten Abstand führt.

8. Arretierbares Gelenk gemäß einem der Ansprüche 5 bis 7, **dadurch gekennzeichnet, dass**
- der Grundkörper (34) einen Zylinder (68') aufweist und der Zylinder (68') eine Zylindereinlassöffnung (66') aufweist,
- der Kolben (70') in dem Zylinder (68') bewegbar aufgenommen ist und die Halteeinrichtung in Form eines Haltebereiches (74') zum Anpressen gegen den Gelenkkopf (30) umfasst, und
- der Haltebereich (74') an der gegenüberliegenden Seite der Zylindereinlassöffnung (66') angeordnet ist.

9. Arretierbares Gelenk gemäß einem der Ansprüche 5 bis 7, **dadurch gekennzeichnet, dass** der Grundkörper (34) eine Gaskartuschenhalterung (38) zum austauschbaren Aufnahmen der Gaskartusche (64) aufweist.

10. Arretierbares Gelenk gemäß einem voranstehenden Anspruch, **dadurch gekennzeichnet, dass**,
- die Halteeinrichtung (74') eine Halteeinrichtung-Berührungsfläche (76') zum Anpressen gegen den Gelenkkopf (30) aufweist, und
- die Halteeinrichtung-Berührungsfläche (76') ringförmig ausgebildet ist und eine Ringbreite von weniger als 1 mm aufweist.

11. Arretierbares Gelenk gemäß einem der Ansprüche 5 bis 10, **dadurch gekennzeichnet, dass** die Halteeinrichtung (74') für ein reibschlüssiges Sperren zwischen der Halteeinrichtung (74') und dem Gelenkkopf (30) angeordnet ist.

12. Arretierbares Gelenk gemäß Anspruch 10 oder 11, wobei die Halteeinrichtung-Berührungsfläche (76') segmentiert ausgebildet ist.

13. Arretierbares Gelenk gemäß Anspruch 11 oder 12, wobei
- der Gelenkkopf einen Gelenkkopf-Außendurchmesser (Dₛₕ) aufweist und in einem ringförmigen Gelenkkopf-Berührungsbereich mit der Halteeinrichtung in Kontakt gelangt, und
- der Berührungsbereich einen Berührungsbereich-Außendurchmesser (D_{ca}) aufweist, der 0,94-mal so groß ist wie der Gelenkkopf-Außendurchmesser (Dₛₕ).

14. Arretierbares Gelenk gemäß einem voranstehenden Anspruch, wobei
- der erste Arm eine Armlängsachse (L) und
- der Berührungsbereich-Außendurchmesser (D_{ca}) und die Armlängsachse (L) einen im Wesentlichen konstanten effektiven Winkel (α) von weniger als 20° bilden.

15. Arretierbares Gelenk gemäß Anspruch 8, wobei
- die Gaskartusche (64) eine Gaskartuschen-Auslassöffnung (62) aufweist,
- der Zylinder (68') und die Gaskartusche eine Gasverbindung über einen Gastransportkanal (61') zwischen der Gaskartuschen-Auslassöffnung (62) und der Zylindereinlassöffnung (66') aufweisen, und
- der Gastransportkanal (61') ein Volumen (V) von weniger als 1000 mm³, insbesondere weniger als 500 mm^{3,} aufweist.

16. Arretierbares Gelenk gemäß einem voranstehenden Anspruch, wobei der Kolben (70') einen Hub (s) von weniger als 2 mm, insbesondere weniger als 1 mm, aufweist.

17. Arretierbares Gelenk gemäß einem voranstehenden Anspruch, wobei der Kolben einen Kolbendurchmesser (D_{Kolben}) von mehr als 20 mm aufweist.

18. Arretierbare Gelenkeinheit, die folgendes aufweist:
(a) ein arretierbares Gelenk (43) gemäß einem voranstehenden Anspruch,
(b) einen zweiten Arm (16) mit einem zweiten Gelenkkopf (84),
(c) eine zweite Gelenkpfanne (88), wobei der zweite Gelenkkopf (84) drehbar in der Gelenkpfanne (88) eingesetzt ist, und
(d) eine zweite Sperrvorrichtung, die zum Sperren des zweiten Gelenkkopfes (84) gegenüber der zweiten Gelenkpfanne (88) eingerichtet ist, und die zweite Sperrvorrichtung folgendes aufweist:
(i) einen zweiten Kolben (70") mit einer trennbaren Gasverbindung zu der Gaskartusche (64), und
(ii) eine zweite Betätigungseinrichtung (86, 50"), die für ein schaltbares Trennen des zweiten Kolbens (70") von der Druckgaskartusche (64) eingerichtet ist.

19. Arretierbare Gelenkeinheit gemäß Anspruch 18, wobei der zweite Arm (16) an der gegenüberliegenden Seite des ersten Arms (14) angeordnet ist.

20. Arretierbare Gelenkeinheit gemäß Anspruch 18 oder 19, wobei die arretierbare Gelenkeinheit folgendes aufweist:
(a) einen Hilfsarm (28) mit einem Hilfsarm-Gelenkkopf (46),
(b) eine Hilfsarm-Gelenkpfanne (42), wobei der Hilfsarm-Gelenkkopf (46) drehbar in der Hilfsarm-Gelenkpfanne (42) eingesetzt ist,
(c) eine Hilfsarm-Sperrvorrichtung (44), die zum Sperren des Hilfsarm-Gelenkkopfes (46) gegenüber der Hilfsarm-Gelenkpfanne (42) eingerichtet ist, und die Hilfsarm-Sperrvorrichtung (44) aufweist:
(i) einen Hilfsarm-Kolben (70) mit einer trennbaren Gasverbindung zu der Gaskartusche (64), und
(ii) eine Hilfsarm-Betätigungseinrichtung (48, 50), die für ein schaltbares Trennen des Hilfsarm-Kolbens (70) von der Gaskartusche (64) eingerichtet ist.

21. Chirurgisches Retraktorgestell, das folgendes aufweist:
(a) eine arretierbare Gelenkeinheit (49) gemäß einem der Ansprüche 18 bis 20,
(b) einen ersten Arm (14), der geeignet ist, einen chirurgischen Retraktor (12) aufzunehmen, und
(c) eine Verankerungseinrichtung (20) zum Festlegen der arretierbaren Gelenkeinheit (49) an einem Operationstisch, und
(d) die Verankerungseinrichtung (20) lösbar an dem Hilfsarm (28) montiert ist.

22. Chirurgisches Retraktorsystem, das ein chirurgisches Retraktorgestell (18) gemäß Anspruch 21 und zumindest einen chirurgischen Retraktor (12), der lösbar an dem ersten Arm (14) montiert ist, aufweist.

23. Verfahren zum Festlegen eines arretierbaren Gelenks (43), insbesondere eines Kugelgelenks, das folgendes aufweist:
- einen ersten Arm (14), wobei der erste Arm einen Gelenkkopf (30) aufweist,
- eine Gelenkpfanne (32), wobei der Gelenkkopf (30) drehbar in der Gelenkpfanne (32) eingesetzt ist,
- eine Sperrvorrichtung, die zum Sperren des Gelenkkopfes gegenüber der Gelenkpfanne eingerichtet ist, und wobei die Sperrvorrichtung folgendes aufweist:
einen Kolben,
eine Gaskartusche, insbesondere eine Kohlendioxidkartusche, und
eine Betätigungsvorrichtung, die für ein schaltbares Trennen des
Kolbens von der Gaskartusche eingerichtet ist,
und die folgenden Schritte aufweist:
- Betätigen der Betätigungseinrichtung, um den Gasdruck am Kolben zu senken, so dass der erste Arm relativ zu dem Pfannengelenk gedreht werden kann, und
- Freischalten der Betätigungseinrichtung, um den Gasdruck am Kolben mittels Freischaltung der Gasverbindung zu der Gaskartusche zu steigern, so dass die Sperrvorrichtung den Gelenkkopf gegenüber der Gelenkpfanne arretiert.

## Revendications

1. Joint verrouillable, en particulier joint verrouillable à rotule et crapaudine, comprenant
(a) un premier bras (14) ayant une tête pivotante (30),
(b) une crapaudine (32), la tête pivotante (30) étant montée en pivotement sur la crapaudine (32), et
(c) un dispositif de verrouillage (90) agencé pour verrouiller la tête pivotante (30) par rapport à la crapaudine (32), le dispositif de verrouillage (90) ayant
(i) un piston (70'),
(ii) une source de gaz sous pression (64), et
(iii) un dispositif d'actionnement (92) agencé pour déconnecter de manière irréversible le piston (70') vis-à-vis de la source de gaz sous pression (64),
**caractérisé en ce que**
(d) la source de gaz sous pression est une cartouche de gaz (64).

2. Joint verrouillable selon la revendication 1, dans lequel la cartouche de gaz (64) présente une pression interne (P_{cartouche}) supérieure à 4 MPa, en particulier supérieure à 6 MPa.

3. Joint verrouillable selon la revendication 1 ou 2, dans lequel la cartouche de gaz (64) contient moins de 1000 g de gaz.

4. Joint verrouillable selon l'une quelconque des revendications 1 à 3, dans lequel la cartouche de gaz (64) contient du dioxyde de carbone liquide, de l'air sous pression, de l'azote sous pression, un oxyde azoté sous pression, ou un mélange de deux ou trois de ces produits.

5. Joint verrouillable selon l'une quelconque des revendications précédentes, dans lequel
- le joint verrouillable (43) a un corps principal (34), le corps principal (34) comprenant la crapaudine (32) et le dispositif de verrouillage (90),
- le dispositif de verrouillage (90) a un élément de fixation (74) qui est reçu dans le corps principal (34), et
- le piston (70') est agencé pour presser l'élément de fixation (74) contre la tête pivotante (30).

6. Joint verrouillable selon la revendication 5, dans lequel le piston et l'élément de fixation (74) sont connectés de telle façon que
- un déplacement du piston (70') dans une direction de travail (P) du piston mène à un mouvement de l'élément de fixation dans une direction de travail (P) de l'élément de fixation ;
- la direction de travail (P) du piston est parallèle à la direction de travail (P) de l'élément de fixation.

7. Joint verrouillable selon la revendication 5 ou 6, dans lequel le piston (70') et l'élément de fixation (74) sont directement couplés, de telle façon qu'un déplacement du piston (70') sur une distance déterminée mène à un mouvement de l'élément de fixation sur la même distance prédéterminée.

8. Joint verrouillable selon les revendications 5 à 7, dans lequel
- le corps principal (34) comprend un cylindre (68'), le cylindre (68') ayant une ouverture d'entrée de cylindre (66'),
- le piston (70') est reçu de façon déplaçable dans le cylindre (68') et comprend l'élément de fixation sous la forme d'une section de fixation (74') pour presser contre la tête pivotante (soit 30), et
- la section de fixation (74') est placée à l'opposé de l'ouverture d'entrée de cylindre (66').

9. Joint verrouillable selon les revendications 5 à 7, dans lequel le corps principal (34) comprend un élément de retenue de cartouche de gaz (38) pour recevoir de façon interchangeable la cartouche de gaz (64).

10. Joint verrouillable selon l'une quelconque des revendications précédentes, dans lequel
- l'élément de fixation (74') est agencé pour presser contre la tête pivotante (30) sur une zone de contact d'élément de fixation (76'), et
- la zone de contact d'élément de fixation (76') a une forme annulaire avec une largeur d'anneau inférieure à 1 mm.

11. Joint verrouillable selon l'une quelconque des revendications 5 à 10, dans lequel l'élément de fixation (74') est agencé pour assurer un verrouillage à friction entre l'élément de fixation (74') et la tête pivotante (30).

12. Joint verrouillable selon l'une quelconque des revendications 10 ou 11, dans lequel la zone de contact d'élément de fixation (76') est segmentée.

13. Joint verrouillable selon les revendications 11 ou 12,
- la tête pivotante possède un diamètre extérieur (Dsh) et est en contact avec l'élément de fixation dans une zone de contact de tête pivotante de forme annulaire, et
- la zone de contact possède un diamètre extérieur (Dca) qui est supérieur à 0,94 fois le diamètre extérieur (Dsh) de la tête pivotante.

14. Joint verrouillable selon l'une quelconque des revendications précédentes, dans lequel
- le premier bras présent un axe longitudinal (L), et
- le diamètre extérieur de la zone de contact (Dca) et l'axe longitudinal du bras (L) forment un angle efficace sensiblement constant (α) inférieur à 20°.

15. Joint verrouillable selon la revendication 8, dans lequel
- la cartouche de gaz (64) présente une ouverte de sortie de cartouche (62),
- le cylindre (68') et la cartouche de gaz sont en communication fluidique avec un conduit à gaz (61') entre l'ouverture de sortie de cartouche (62) et l'ouverture d'entrée de cylindre (66'), et
- le conduit à gaz (61') possède un volume (V) inférieure à 1000 mm³, en particulier inférieur à 500 mm³.

16. Joint verrouillable selon l'une quelconque des revendications précédentes, dans lequel le piston (70') présente une course (s) inférieure à 2 mm, en particulier inférieure à 1 mm.

17. Joint verrouillable selon l'une quelconque des revendications précédentes, dans lequel le piston a un diamètre (Dₚᵢₛₜₒₙ) supérieur à 20 mm.

18. Ensemble formant joint verrouillable, comprenant
(a) un joint verrouillable (43) selon l'une quelconque des revendications précédentes,
(b) un second bras (16) ayant une seconde tête pivotante (84),
(c) une seconde crapaudine (88), la seconde tête pivotante (84) étant montée en pivotement sur la seconde crapaudine (88), et
(d) un second dispositif de verrouillage agencé pour verrouiller la seconde tête pivotante (84) par rapport à la seconde crapaudine (88), le second dispositif de verrouillage ayant
(i) un second piston (70") en communication fluidique capable d'être déconnecté avec la cartouche de gaz (64), et
(ii) un second dispositif d'actionnement (86, 50") agencé pour déconnecter de manière réversible le second piston (70") vis-à-vis de la cartouche de gaz sous pression (64).

19. Ensemble formant joint verrouillable selon la revendication 18, dans lequel le second bras (16) est agencé à l'opposé du premier bras (14).

20. Ensemble formant joint verrouillable selon l'une quelconque des revendications 18 ou 19, comprenant
(a) un bras de support (28) ayant une tête pivotante (46) sur le bras de support,
(b) une crapaudine (42) sur le bras de support, la tête pivotante (46) étant montée en pivotement sur la crapaudine (42) du bras de support,
(c) un dispositif de verrouillage de bras de support (44) agencé pour verrouiller la tête pivotante (46) par rapport à la crapaudine (42) du bras de support, le dispositif de verrouillage de bras de support (44) ayant
(i) un piston de bras de support (70) en communication fluidique capable d'être déconnecté avec la cartouche de gaz (64),
(ii) un dispositif d'actionnement de bras (48,50) agencé pour déconnecter de façon réversible le piston de bras de support (70) vis-à-vis de la cartouche de gaz (64).

21. Cadre rétracteur chirurgical, comprenant :
(a) un ensemble formant joint verrouillable (49) selon l'une quelconque des revendications 18 à 20,
(b) le premier bras (14) étant adapté à recevoir un rétracteur chirurgical (12), et
(c) un élément d'ancrage (20) pour monter l'ensemble formant joint verrouillable (49) sur une table d'opération,
(d) l'élément d'ancrage (20) étant monté de façon libérable sur le bras de support (28).

22. Système rétracteur chirurgical, comprenant le cadre rétracteur chirurgical (18) selon la revendication 21, et au moins un rétracteur chirurgical (12) monté de façon libérable sur le premier bras (14).

23. Procédé pour fixer un joint verrouillable (43), spécialement un joint verrouillable à rotule-et-crapaudine, ayant
- un premier bras (14), le premier bras ayant une tête pivotante (30),
- une crapaudine (32), la tête pivotante (30) étant montée en pivotement sur la crapaudine (32),
- un dispositif de verrouillage agencé pour verrouiller la tête pivotante par rapport à la crapaudine, le dispositif de verrouillage ayant un piston,
une cartouche de gaz, en particulier une cartouche de dioxyde de carbone, et
un dispositif d'actionnement agencé pour déconnecter de façon réversible le piston vis-à-vis de la cartouche de gaz,
comprenant les étapes consistant à :
- actionner le dispositif d'actionnement, supprimant ainsi la pression sur le piston de telle façon que le premier bras peut être pivoté par rapport à la crapaudine, et
- libérer le dispositif d'actionnement, mettant ainsi le piston sous pression en l'amenant en communication fluidique avec la cartouche de gaz de telle façon que le dispositif de verrouillage verrouille la tête pivotante par rapport à la crapaudine.
